# EUROPEAN PATENT APPLICATION

(11) **EP 1 835 288 A1**
(43) Date of publication of application: **19.09.2007**
(21) Application number: 07386003.3
(22) Date of filing: 06.02.2007
(51) Int. Cl.: G01N 33/68

(54) **Method for the analysis of naturally occuring peptides and small proteins in cells and solid biological materials using mass spectrometry**

(30) Priority: 09.02.2006 GR 20060100080
(71) Applicant: Foundation for Biomedical Research of the Academy of Athens, 115 27 Athens (GR); Tsangaris, George, 152 35 Vrilissia Attikis (GR); Vougas, Constantinos, 15669 Papagou (GR)
(72) Inventor: Tsangaris, George, 152 35 Vrilissia Attikis (GR); Vougas, Konstantinos, 156 69 Papagou (GR)
(74) Representative: Tefou-Fotea, Eleni

(57) **Abstract**

One method was developed for the separation and identification of the naturally occurring peptides and small proteins (NOP/SP) present in solid biological material with the use of mass spectrometry. The separation of protein molecules with molecular weight <10kDa is achieved through mixing and homogenisation of the solid biological materials with aqueous solution containing 50% acetonitrile and 0.1% trifluoroacetic acid (TFA). As opposed to the methods currently used, the present method does not include complicated and time consuming steps (fractionation, chromatography, ultrafiltration, etc.). Because of the NOP/SP analysis with MALDI-TOF the method is rapid, subject to automation and quantification. These elements constitute the method as highthrouput and appropriate for pathological states diagnosis, either through analysis of one or more NOP/SPs as specific biological markers, or through comparison of normal and pathological patterns/profiles.

## Description

### B. TECHNICAL FIELD WHERE THE INVENTION IS ADDRESSED

This invention refers to a method for the separation and identification - by means of mass spectrometry - of peptides and small proteins sized 1.000-10.000Da (1-10kDa) referred to as Naturally Occurring Peptides and Small Proteins (NOP/SP) present in solid biological material such as tissues, biopsies, surgery samples etc and in cells which can be either isolated from tissues (such as hepatocytes, splinocytes) and biological fluids (such as peripheral blood lymphocutes, amniocytes etc) or from cell in cultures. By application of this method NOP/SP are separated from the proteins with Molecular Weight greater than 10kDa present in solid biological materials and cells, without complicated and time consuming procedures and analyzed *per se* by mass spectrometry. This method provides the possibility for the analysis of the NOP/SP using MALDI-TOF mass spectrometry, which is fast and can be potentially automated and the analysis can be further enhanced by means of NOP/SP quantitation. All these facts render this method suitable for the analysis of a great number of samples and for application in the diagnostic field, either through qualitative or quantitative analysis of one or more NOP/SP in the context of specific biological markers indicative of disease or in the context of protein profile comparison between normal and diseased samples.

### C. UTILITY OF THE AFOREMENTIONED TECHNIQUE

### C.1. INTRODUCTION

### C.1.1. PROTEOMICS

The term «proteomics» was initially indicted in 1996 by Marc Wilkins and is originating from the abbreviation of the words protein and genome. This term describes the sum of the protein content of a biological system at a given time or state. Since that time methodologies and techniques such as bioinformatics and mass spectrometry allowing the analysis of proteins have evolved rapidly. Some of them such as 2-D polyacrylamide gel electrophoresis (2D-PAGE) have come to be especially popular. Proteomics have been widely utilized in the academic field as well as within the pharmaceutical industry sector since there is a strong belief that it will be a leading research factor in the post-genomic era.

The main field of proteomics is the full analysis-mapping of the protein content of a sample. The established and general approach of the proteomic analysis includes four steps: protein isolation from the sample, protein separation, protein detection and finally protein identification. Apart from the aforementioned analytical techniques, the protein identification procedure includes specific software for image analysis of polyacrylamide gels which includes gel comparison, in-gel protein quantitation and data storage and management.

### C.1.2 PROTEOMIC METHODOLOGY

### C.1.2.A. Protein separation

The methodology applied for the protein isolation from a sample is sample dependant. It also depends on the procedure that will be used for protein separation and identification. In the case which the protein separation will occur with the well established and general approach of isoelectric focusing, the protein isolation end up in the proteins' solubilisation in a specific denaturation solution which mainly contains Urea up to 8M concentration and additionally could contain Theiourea, CHAPS, 1,4-DTT, Tris, EDTA and protease inhibitors. In the case of biological fluids the proteins are already in solution. The sample is centrifuged in order all the insoluble cellular material and other insoluble elements to be removed. Following that, sample containing 1 mg of total protein is diluted in an appropriate volume of the aforementioned denaturation solution prior to further treatment. Due to the fact that the protein concentration is dependent upon the biological fluid type, in the case of very dilute biological fluids such as amniotic fluid or urine the samples are concentrated after the centrifugation and prior to the dilution with the denaturation solution. Sample concentration can be achieved using protein precipitation, ultrafiltration etc.

In the case of solid biological materials (tissue, cells, biopsies etc.) the proteins need to be extracted into the solution prior to any further steps. This is achieved through homogenization (mechanical pressure, ultrasound etc.) in a small volume either of the aforementioned denaturation solution or of other types of solutions. After that, the already mentioned steps of centrifugation and dilution in denaturation solution take place in order the sample to be prepared for further analysis.

Finally, for the isolation of specific protein groups from a biological sample, specific fraction techniques such as chromatography or immunoprecipitation can be applied prior to further treatment.

### C.1.2.B. Protein separation using two dimensional acrylamide gel electrophoresis (2D-PAGE).

The general and classic method for the protein separation and detection used nowadays in proteomics is 2D SDS PAGE Gel Electrophoresis. The method is based on the concurrent isolation of proteins on the basis of their electric charge and molecular weight. The method results in the separation and visualization of hundreds of proteins at once, which is something that no other method is currently able to achieve. Initially the technique includes the isoelectric focusing (IEF) of proteins, meaning protein separation based on their electric charge, and finally their molecular weight based separation.

IEF is literally an equilibration process, during which proteins are forced to move along a strip carrying acrylamide derivatives (immobilines) of differential pH (IPG strip) under the effect of a powerful electric field. The proteins move and focus towards the points of the strip where their charge is neutralized (isoelectric point). There are IPG strips that cover a wide range of pH units such as pH 3-10 (length 7-24cm) as strips that are suitable for the analysis of very narrow pH ranges such as pH 3.5-4.5). One of the advantages of the IPG strips is the relatively large amount of protein sample that can be analyzed. The exact conditions of the IEF are dependant on a number of factors such as the quantity of the sample to be analyzed, sample application on the IPG strip, electric potential and isoelectric focusing duration.

The protein separation based on their molecular weight as a second dimension is achieved through the use of a polyacrylamide gel. This gel can be of constant or graded acrylamide concentration depending on the expected molecular weight of the proteins under study. The most commonly used gels are of constant concentration of 12% acrylamide and the gradient acrylamide concentration 9-16% for the separation of proteins of molecular weight range 10-200kDa.

### C.1.2.C. Protein Detection

After the protein separation in the acrylamide gel, proteins are detected through gel staining using sensitive stains the choice of which depends on the identification procedure that follows. The stains most commonly used are coomasie blue and silver stain. These stains are chosen when the mass spectrometry is going to be used for protein identification.

Given the proteinic heterogeneity in the 2D gels, the proteins are usually represented by more than one spots resulting in a number of translated gene products far greater than the number of the actual genes encoding them. In eukaryotes has been observed that it is possible for a single gene to encode 5-20 different proteins.

### C.1.2.D. Mass Spectrometry

The high throughput identification of proteins is achieved by mass spectrometry. Protein spots are removed mechanically from the 2D gel, they are distained, treated with trypsin (in-gel) and the resulting peptides are extracted into solution. The extracted peptides are analyzed by mass spectrometry for their mass and amino acid sequence to be determined.

Mass spectrometry comprises several steps for producing the desired measurements such as peptide ionization, release of these ionized peptides into the gas phase and high accuracy measurement of their mass. The ionization process and the release of the ions in the gas phase can be achieved with : a) Matrix Assisted Laser Desorption lonisation (MALDI), b) Surface Enhanced Laser Desorption lonisation (SELDI) and c) Electrospray lonisation (ESI). Following that the mass measurement can be achieved by: a) Measurement of the time of flight of the ions in a high vacuum tube (TOF), b) using an ion trap (IT), c) using quadrapoles and d) using a Fourier Transform system. Combining the ionization and mass measurement technologies the following techniques, which are widely used nowadays, accrue: a) MALDI-TOF, b) SELDI-TOF, c) ESI-TOF, d) ESI-IT, e) ESI-FT, f) ESI-IT-FT, g) ESI-QqTOF, h) ESI-QqQ etc.

According to the aforementioned techniques the peptide mass determination occurs by measuring the time of flight of the peptides in high vacuum tubes, while further analysis includes the deduction of the amino acid sequence of these peptides. Using highly specialized software the mass of the detected peptides is compared with the theoretical mass of all the peptides resulting from all the proteins of all known organisms which are indexed in various proteomic and genomic database and the protein identification is derived using highly sophisticated probabilistic models. This identification is usually based on more than five peptides which can be more that 20 in several instances, especially when the protein under study is of high molecular weight, while the probability of an error being less than 10⁻⁶, depending on the number of peptides that where used for the protein identification. Even more reliable results can be acquired by deriving the amino acid sequence of the analyzed peptides through the techniques of MALDI-MS-MS and ESI-MS-MS. In that instance the protein identification occurs through the amino acid sequence of the peptides and the final chance of an error is less than 10⁻¹⁰ depending on the number pf peptides analyzed for the specific protein.

From the aforementioned techniques MALDI-TOF and SELDI-TOF are characterized as high throughput because of their ability to rapidly and reliably analyze hundreds of samples. Because of the fact that the MALDI-TOF technology does not require peptide separation, is especially sensitive to organic and inorganic contaminant ions. Although the MALDI-TOF technology has a very high resolving power combined with great sensitivity in the signal detection the presence of contaminants result in a high level of mass spectra background noise which reduce the significance of the collected signal. In order for the mass spectra being produced from the MALDI-TOF technique to be evaluated the peptide mixture crystallized on the steel target has to be free of contaminant to the extent that is possible and the quantity of the peptides that go into the gas phase to be at least two times more intense than the background noise (Signal/Noise=2).

The SELDI-TOF technique, concerning the separation of the molecules under analysis, is based on the affinity of those molecules to specific substrates (biochip). For this reason a great number of different stationary sorbents (hydrophobic, anionic cationic and metallic) are used aiming at the specific binding of proteins of interest on their surface. The main disadvantage of this method is that the instrument that is used for the mass measurement is of low resolving power and not sensitive enough to detect lots of important molecules that are present in very small amounts in the sample. Furthermore the specific (low abundance) molecules are likely not to adhere to the biochips which are used in the SELDI-TOF technique, but to be washed of the sorbents surface along with other molecules, thus losing valuable information. Finally the ESI techniques have the advantage of unambiguous protein identification through amino acid sequencing but are extremely time consuming since prior to mass measurement there is a sample fractionation step using liquid chromatography. This fractionation step causes the analysis duration for a single sample to be between 25 minutes to 3 hours, while using the MALDI-TOF or the SELDI-TOF technologies, 384 samples are being processed within 2 - 3 hours.

In proteomics all the aforementioned techniques can be used simultaneously and in a complementary way in order all the valuable information concerning proteome differentiation in a disease state in relation to a normal state to be retrieved. The identification of these differentiations can be used as biological markers and therapeutic targets.

### C.2. The proteome of the solid biological materials

The biological materials can in principle be separated in three categories: **a) biological fluids** which include peripheral blood, urines, cerebrospinal fluid, amniotic fluid etc **b) solid biological material** which include tissue sections, cells, bones etc. and **c) biological gas** which included the gases present in the peripheral blood etc.

The solid biological material section includes any biological material, being in solid state, which can be collected from an organism in normal or pathological states. The term solid biological material includes a big group of materials such as tissues and the individual cells comprising them, the bones, the cellular component of the biological fluid (e.g. peripheral blood lymph cells, amniotic fluid cells etc.), cells grown in vitro or ex-vivo in cell cultures etc. The solid biological materials can be obtained from an organism either by invasive methods such as biopsy or surgery, or through non-invasive techniques. The solid biological material both at the tissue and cellular level, contain a huge number (approximately 100.000) different protein molecule (proteins, peptides, oligopeptides, polypeptides etc.). Because of this diverse protein content, it is certain, that the solid biological materials contain information relevant to the normal or pathological conditions that take place at the cellular and tissue level. The identification of various proteins and protein types in such materials could therefore provide valuable insight in the way the cells and tissues interact with their environment and react to various normal or pathological conditions. Such knowledge would contribute to our more thorough understanding of the underlying mechanisms of health and disease providing at the same time new therapeutical targets.

Given all the aforementioned facts, solid biological material is currently considered a "multipotent hot topic" of proteomic research focusing mainly on the discovery and identification of biological markers and active molecules which will aid in the more thorough understanding, diagnosis and cure of the disease. The solid biological material proteomic analysis is a tedious and complicated task primarily because of the great number of proteins present in the sample and additionally because of the great diversity in concentration of the different protein species. In the case of tissue proteomic analysis the complexity is increased because of the fact that a tissue is comprised by different cellular species e.g. brain.

Relevant studies indicate that 60-70% of the solid state biological material proteome is comprised of the high abundance proteins which are mainly structural (actin, tubulin etc.). The remaining proteome includes the low abundant proteins which seem to be the proteins presenting the greatest interest since they are directly related to the phenotype of each biological material.

The low abundance part of the proteome is divided into three groups, the high molecular weight proteome which include the proteins with a Molecular Weight greater that 70kDa, the medium molecular weight proteome which includes proteins with molecular weight in the range of 30 - 70 kDa and the low molecular weight proteome which includes proteins with molecular weight less than 30kDa.

The vast molecular variation of the low abundant proteins present in solid state biological material containing many different cell types plus their low abundance make their detection and analysis a very tedious task. Several approaches have been adopted to tackle this problem such as fractionation, separation and concentration of the biological sample. The greatest problem is caused by the high abundant proteins since their presence is masking the low abundance proteins inhibiting their detection. The obvious solution would be the removal of these high abundant proteins. Within that experimental frame several techniques have been utilized primarily aiming at the removal of albumin and immunoglobulins such as Cibacron dye, A/G Columns, antibodies, molecular weight cut-off filters, affinity chromatography, size exclusion chromatography etc. All these techniques have made the detection of the low abundant proteome possible. Nevertheless a very important fact needs to be considered. Albumin and other high abundance proteins such as a-2 macroglobulin are carrier proteins. This means that they bind and carry a great number of low abundant proteins. The removal of these carrier proteins would also mean the removal of all the proteins which are being carried, most of which are low abundant and of extreme interest. For instance the antimicrobial peptide a-defensin circulates in plasma being bound to albumin, a-2 macroglobulin and several serpins and is removed during high abundant protein depletion procedures.

### C.3. The low molecular weight proteome of the solid biological materials.

The solid biological material low molecular weight proteome includes a huge variety of protein molecules and peptides with molecular weight less than 30kDa. The low molecular weight proteome includes the Small Proteins (SP) with molecular weight more than 10kDa and the Naturally Occurring Peptides (NOP) with molecular weight less than 10kDa. The Naturally Occurring Peptide fraction includes peptides coming from protein degradation, processing of immature protein precursors and from the post-transcriptional intra and extra cellular protein alterations. All these processes operate within the complicated biological systems, offering additional functionality to the protein molecules, since the intact protein as well as its fractions can be utilized in many different metabolic pathways and cellular processes. A typical example of the above statement is the gene of pro-opio-melanocortin and the precursor protein that is produced by it, which after the post-translational, intracellular processing produces ten different peptidic hormones. Naturally Occurring Peptides and Small Proteins (NOP/SP) drive through tissues and cellular formations easily because of their small size and diffuse thereafter in the peripheral blood. Additionally, since these peptides are relatively resistant to proteolytic degradation they have a long half-life. These two characteristics render Naturally Occurring Peptides easily accessible and detectable, therefore good candidates for biological markers. Relevant studies in the human plasma and serum have illustrated that small protein molecules are directly related to diseases such as cancer, diabetes and cardiovascular diseases as well as other infectious diseases. More specifically, using the SELDI-TOF MS in conjunction with bioinformatic tools for protein profile comparison, five protein molecules were detected that were directly related to ovarian and prostate cancers. Four of these protein molecules had a molecular weight less that 2.500 Da and the last one had a molecular weight of 3.080 Da. These finding imply that the solid state biological material low molecular weight proteome contains a very important deposit of histological information and can provide useful biological markers for the diagnosis of various diseases.

### C.3.A. Methods for the separation, detection and identification of the naturally occurring peptides and small proteins in biological fluids.

Naturally Occurring Peptides and Small Proteins (NOP/SP) belong to the low molecular weight proteome of the low abundant protein fraction of biological fluids. Furthermore these molecules are characterized by small molecular weights (<10kDa) and cannot be separated detected and identified with the classic wide use proteomic techniques as described in Chapter C.1.2. since the molecular weight range of the proteins that can be analyzed with these techniques fluctuates between 10 and 200kDa. There are several techniques that have been used for the identification of proteins belonging to the low molecular weight proteome of the biological fluids, and mainly include various low abundant protein enrichment schemes in conjunction with mass spectrometric techniques. Even though these techniques are not solely used for the analysis of naturally occurring peptide and small proteins, they can successfully identify these molecules among other of higher size. The methods currently used can be separated in three major groups: a) the low molecular weight proteome is separated by centrifugal ultrafiltration using filters of specific molecular weight cut-off, b) the low molecular weight proteome is separated-enriched by sample treatment with 2 volumes of pure acetonitrile and c) the naturally occurring peptides and small proteins of the plasma or the blood serum are fractionated in 96 fraction by liquid chromatography and are subsequently analyzed with mass spectrometry. In all the case the identification of the proteinic molecules is achieved via the combined use of liquid chromatography and mass spectrometry. Because of the last fact none of these methods are high throughput. Furthermore, since the concentration of the naturally occurring peptides and small proteins is quite low, incorporating multiple processing steps can result in loss of valuable information.

Regarding the naturally occurring peptides and small proteins identification in biological fluids, an original method has been developed. This method constitutes the analysis and identification of these molecules by mass spectrometry possible. A patent application for this method has already been submitted (Patent application Number: 20050100392/27-7-2005).

### C.4. Relevant Bibliography

**1.** Characterization of the low molecular weight human serum proteome. Tirumalai RS, Chan KC, Prieto DA, Issaq HJ, Conrands TP, Veenstra TD. Mol Cell Proteomics 2003, 2: 1096-1103.
**2.** Low-molecular-weight human serum proteome using ultrafiltration, isoelectric focusing, and mass spectrometry. Harper RG, Workman SR, Schuetzner S, Timperman AT, Sutton JN. Electrophoresis 2004, 25: 1299-1306.
**3.** Top-Down analysis of the low molecular weight human plasma proteome using hybrid Ion Trap-Fourier Transform Mass Spectrometry. Sutton JN, Bonilla LE, Richmond T, Gerszten RE, Liu E, Shi X, Senko M, Zabrouskov V, Kelleher N, Forbes A, Harper RG. Thermo Electron Corporation, Application Note: 344, 2005.
**4.** Analysis of low-abundance, low-molecular-weight serum proteins using mass spectrometry. Merrell K, Southwick K, Graves SW, Esplin MS, Lewis NE, Thulin GD. Journal of Biomolecular Techniques 2004, 15: 238-248.
**5.** USA Patent number: US4171204/1979.
**6.** Organic solvent extraction of proteins and peptides from serum as an effective sample preparation for detection and identification of biomarkers by mass spectrometry. Chertov O, Biragyn A, Kwak LW, Simpson JT, Boronina T, Hoang VM, Prieto DA, Conrads TP, Veenstra TD, Fisher RJ. Proteomics 2004, 4: 1195-1203.
**7.** Method for the analysis of naturally occurring peptides and small proteins in biological fluids using mass spectrometry (Patent Application Number: 20050100392/27-7-2005).

### D. Presentation of the invention as it is defined in the claims.

The present invention refers to a method for the treatment of solid state biological material, biopsy samples and cells as obtained from cell cultures, tissues (hepatocytes) and biological fluids (e.g. peripheral blood lymphocytes, amnion fluid cells), which aims at the isolation and identification of the Naturally Occurring Peptides and Small Proteins (NOP/SP) of molecular weight less than 10kDa using mass spectrometry. NOP/SP as already mentioned belongs to the low abundance low molecular weight proteome of the solid biological material. Apart from the low abundance of these molecules an additional problem for their detection is the fact that in solid state biological material the small protein molecules are often attached to high molecular weight high abundance carrier proteins such as albumin. The NOP/SP isolation method has to facilitate their release in solution from these carrier proteins.

For the method development of the current invention the fact that the protein solubility is a result of polar interactions with the soluble face, ionic interactions with salts and electrostatic forces between charged molecules bearing the same charge, was taken into consideration. The disturbance of these interactions with the use of organic solvents, acids, salts or metallic ions causes the decrease of protein solubility and finally their precipitation from the solution containing the solid state biological material total protein content as derived from the homogenization of the solid biological material. The organic solvents, when used as protein precipitants, have double action. They decrease the protein solution dielectric constant, fact that favors the electrostatic interactions between protein molecules. Furthermore, they displace water molecules from the protein surface which causes the electrostatical interactions of hydrophobic interactions to prevail, causing protein aggregation and precipitation. Additionally proteins present their minimum solubility at their isoelectric point because of the fact that there is no net electric charge on the protein molecule. It has also been observed that acidic reagents cause the formation of insoluble salts with the positively charged protein amino groups in environments where the pH is less than their isoelectic point and thus low pH favors protein precipitation. Relevant studies have shown that the addition of pure acetonitrile in plasma in a volumetric ratio of 2:1 (2 volumes of acetonitrile to 1 volume of sample) precipitates a minimum of 92% of the total protein amount present while the use of acidic reagents such as TFA and formic acid not only has been shown to facilitate the protein precipitation but also seems to have an effect in the protein ionisation during their further analysis with LC-MS. [Optimization of protein precipitation based upon effectiveness of protein removal and ionisation effect in liquid chromatography-tandem mass spectrometry. Polson C, Sarkar P, Incledon B, Raguvaran V, Grant R. Journal of Chromatography B 2003, 785: 263-275].

In a relevant patent (Application Number: GR20050100392/27-7-2005, Patent No: 1005304/27-7-2005), 50% acetonitrile 0.1%TFA aqueous solution has been used for the selective separation and identification of the naturally occurring peptides and small proteins (NOP/SP) in biological fluids.

Taking all the above into consideration, in the current invention acetonitrile was used for the separation of NOP/SP from the rest of the proteins present in the solution containing the total protein content of solid biological materials with MW>10kDa, primarily because of its effectiveness as protein precipitant and additionally because of its ability to disrupt protein-protein bonds, for NOP/SP to be released in solution. In contradiction to the up-to-date methodologies for the analysis of the low molecular weight proteome of the solid biological materials, this invention's originality lies to the fact that the homogenization of the solid state biological material (either cells or tissue sections) is carried out with the use of aqueous solution containing 50% acetonitrile and 0.1 % TFA. By that procedure NOP/SP were separated for the remaining protein molecules of the solid state biological materials with molecular weight greater that 10kDa and their analysis as naturally occurring molecules using mass spectrometry became possible.

### D.1. Method Presentation

The method described in the current invention is comprised of three stages: A) Sample preparation, B) NOP/SP selective separation and C) NOP/SP analysis using mass spectrometry.

### D.1.A. Sample Preparation

### 1) Tissue, tissue section, biopsy etc.

Full tissue, tissue section, biopsy sample and other solid state biological materials can be treated (D.1.B) for NOP/SP analysis. Prior to this treatment, washing of the solid state biological material with the appropriate solution (normal saline, phosphate buffers etc.) is possible.

### 2) Cells surviving in cell cultures

For cells surviving in cell cultures under in-vitro or ex-vivo conditions, in the case the cells are in suspension a volume of the culture containing the appropriate number of cells is centrifuged in order for the cells to precipitate. In the case of the attached cells, the growth medium is disposed and the attached cells are separated from the adhesion matrix using appropriate methods (mechanical, enzymatic, etc.). The suspension containing the detached cells is centrifuged for the cells to precipitate. In all the cases the supernatant is disposed and the cell pellet is treated as described in the following section (D.1.B) for NOP/SP analysis. The aforementioned procedure can be applied for every cell-type that survives and is studied under in vitro or ex-vivo conditions. Additionally, prior to treatment for NOP/SP analysis the cell pellet can be resuspended and washed using appropriate solutions such as normal saline, phosphate buffers, etc.

### 3) Cells originating from biological fluids

In the case of cells originating from biological fluids, the cells under study are isolated from the biological fluid using an appropriate method (simple centrifugation, centrifugation using a density gradient, flow cytometry, magnetic beads, etc.) and is treated according to section D.1.B for NOP/SP analysis. Additionally prior to treatment for NOP/SP analysis the cell pellet can be resuspended and washed using appropriate solutions such as normal saline, phosphate buffers, etc.

### 4) Cells and/or specific cell type originating from a tissue or a tissue section.

In the case of NOP/SP analysis of a specific cell type originating from a tissue or tissue section, the tissue is processed in order for the different cellular types comprising the tissue to be separated. Following that, the specific cellular type under study is isolated with the use of an appropriate method (centrifugation, use of antibodies against cell-surface antigens, flow cytometry, use of magnetic beads etc.) and is treated as described in section D.1.B. for NOP/SP analysis. In the case of non-solid tissues such as bone marrow, the isolation of the specific cellular type under study can be performed directly on the tissue by application of a suitable method (centrifugation, use of antibodies against cell surface antigens, flow cytometry, use of magnetic beads etc.). In the case of fixed tissues (parafin sections, cryo sections etc.) the areas or cells of interest are isolated by an appropriate technique such as laser capture microdisection. Following that the sample is treated for NOP/SP analysis (see section D.1.B). Additionally, the obtained cells or tissue section can be washed with an appropriate solution such as normal saline or phosphate bufffer and be re-precipitated (centrifugation) prior to treatment for NOP/SP analysis.

### D.1.B. Sample treatment for NOP/SP selective preparation.

In a quantity of a solid biological material (tissue, tissue section, cell pellet, cells originating from biological fluids etc.) obtained according to appropriate sample treatment procedure as described in section D.1.A., an appropriate volume of **50% acetonitrile, 0.1% Trifluoroacetic acid (TFA) aqueous solution** is added. The mixture is vortexed for 5-10sec and them it is homogenized by application of one of several available methods (ultrasound, mechanical homogenization, freeze thaw cycles in liquid nitrogen etc.) or even by combining two or more methods. Following the homogenization the solution is centrifuged at 13.000rpm (15.000g) for 30 minutes at 4°C. The supernatant is transferred to a new tube and is concentrated by centrifugation under vacuum up to an appropriate volume for the followed stages of NOP/SP analysis.

### D.1.C. NOP/SP analysis using mass spectrometry

Prior to analysis with mass spectrometry, the concentrated sample is desalted with the use of a pipette tip packed with C18 material. Prior to the desalting of the concentrated sample, the C18 material packed in the tip is washed with pure acetonitrile HPLC grade, then with 50%acetonitrile in water and is finally equilibrated with aqueous solution containing 3% acetonitrile and 0.1%TFA. After the equilibration step the concentrated sample passes through the material several times by pipetting up and down with an appropriate pipette. At that step the NOP/SP are bound on the C18 material. The sample is desalted by further washes with 3% acetonirile, 0.1 %TFA. Finally NOP/SP are eluted from the C18 material using aqueous solution 60% acetonitrile, 0.3%TFA directly on a steel MALDI target. Matrix solution [0.3% (w/v)a-Cyano-4-hydroxycinamic acid, 50% (v/v)acetonitrile, 0.1%(v/v)TFA] is then added to the eluant and the mixture is allowed to dry. After the solution has dried and a spot has formed, matrix solution is again added and is allowed to dry. Following that procedure the MALDI target is inserted in the MALDI instrument for the analysis. Alternatively the eluant from the C18 material is placed on a specific MALDI target spot were matrix solution has previously been allowed to dry. After drying of the eluant matrix solution is again added and after it dried the target is placed in the instrument for the analysis.

For the analysis of the concentrated sample using an ESI-TOF mass spectrometer, the sample is desalted using pipette tip packed with C18 material using the previously described procedure the only difference being that instead of 0.1%TFA, 5% formic acid is used in all the solutions. Consequently, the NOP/SP are eluted using aqueous solution 60% acetonitrile 5% formic acid and are collected in an eppendorf. A fraction from the eluant is then placed in a special glass tube with external metallic coating and small cross-section and is injected-infused into the mass spectrometer with pressure and application of high voltage.

### D.2. Method Effectiveness

For the evaluation of the effectiveness of the method that is described in the current invention regarding the analysis of NOP/SP, the following experiments were performed.

### a) NOP/SP selective separation

Cell culture of the T-cell line CCRF-CEM containing 20x10⁶ cells is treated as described in Section D.1.A.2. and the cells washed twice with normal saline. For NOP/SP isolation, the cell pellet is being treated as described in section D.1.B. In particular the cell pellet is resuspended in 200µl of 50% acetonitrile, 0.1% TFA aqueous solution. The mixture is homogenized by ultrasound, and then is centrifuged in order for the pellet and supernatant to be separated. The supernatant is collected and concentrated using centrifugation under vacuum up to the volume of 25µl. In the pellet as well as in the concentrated supernatant, denaturation solution containing 20mM Tris, 7M Urea, 2M Theiourea, 4% CHAPS, 10mM 1,4DTE, 1mM EDTA, 1mM PMSF, protease inhibitors, 0.2mM Na₂VO₃ and 1mM NaF is added up to a final volume of 250µl. A cell culture grown in parallel with the first one containing exactly the same number of cells is treated according to the classic proteomic methodology (C.1.2.). In particular the cell culture is treated as described in Section D.1.A.2. and is washed twice with normal saline. 250µl of the denaturation solution are added to the cell pellet. The cells are homogenized with ultrasound, the solution is centrifuged and the supernatant collected.

Initially the protein material of these samples is subjected to isoelectric focusing in strips with a non-linear pH range of 3-10 (first dimension). The focusing begins with 250V for 30 minutes and then it rises in a linear way within 14 hours to 6000V. At that point it remains constant for another 18 hours. After the end of the isoelectric focusing the proteins are separated on the basis of their molecular weight (second dimension). The IPG strips carrying the focused proteins is applied to a 12% acrylamide gel. These gels run for 6 hours at 15Watt/gel. After the end of the electrophoresis the gels are fixed in 50%methanol, 5% phosphoric acid for 2 hours and then it is stained with Colloidal Coomassie for 16hours.

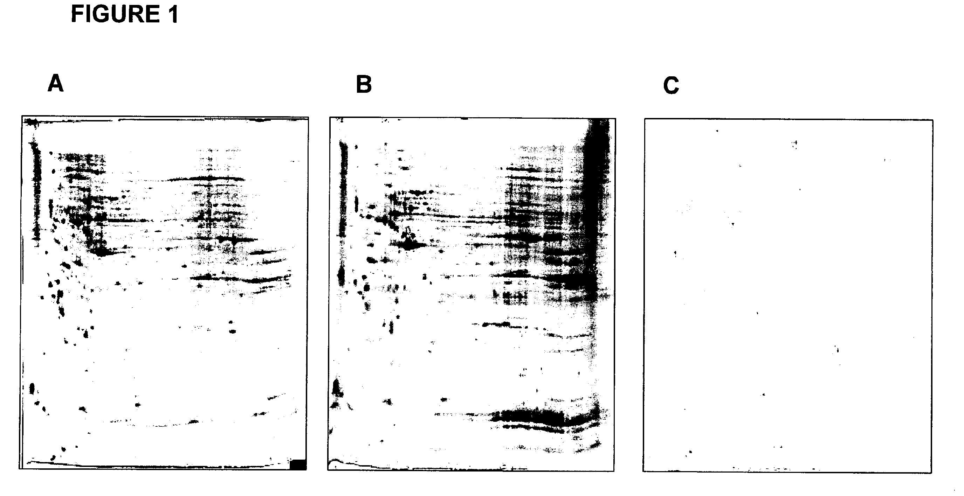

From the comparison of the gels in FIGURE 1 it is ascertained that the application of the method as described in Section D.1.B. results in the precipitation of most of the proteins with molecular weight greater than 10kDa. In particular it is evident that the CCRF-CEM cells homogenization in the 50% acetonitrile 0.1% TFA aqueous solution resulted in the precipitation of the proteins with molecular weight >10kDa leading to the selective enhancement of the NOP/SP presence in the solution (FIGURE 1 B). Since NOP/SP remain in solution, their further analysis is possible (FIGURE 1C).

### b) Efficiency of the acetonitrile/TFA solution.

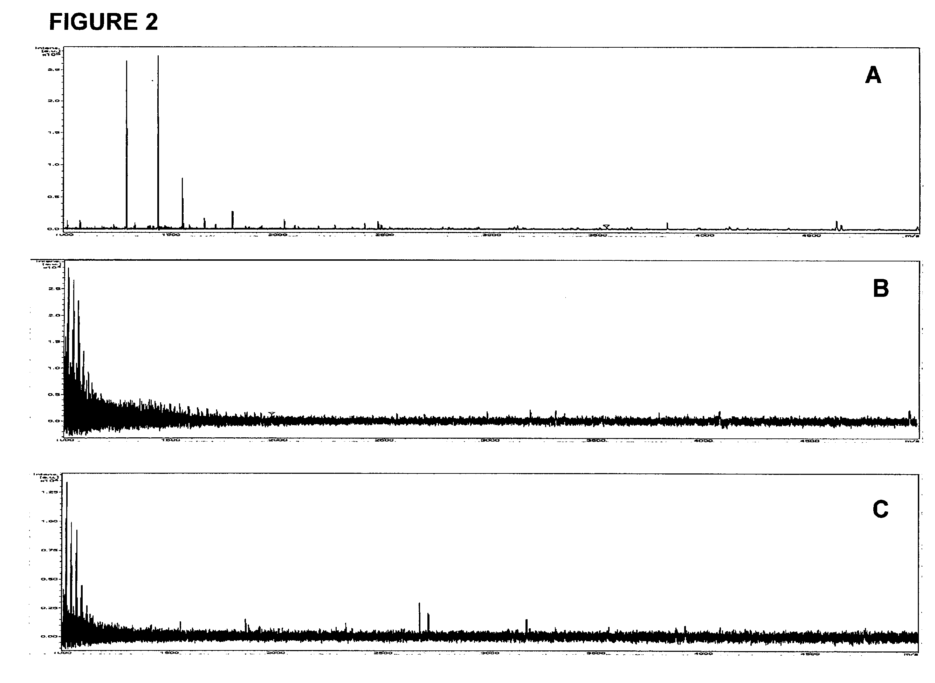

For the enhancement of acetonitrile/TFA solution efficiency, regarding the NOP/SP selective separation from the rest of the solid biological material proteins with molecular weight >10kDa the following experiments were carried out:
**1)** A T-Cell line CCRF-CEM cell culture containing 20×10⁶ is treated with 200µl acetonitrile/TFA solution as described in sections D.1.A.2 and D.1.B. NOP/SP are analyzed by MALDI-TOF mass spectrometry as described in section D.1.C. The corresponding spectrum is displayed in FIGURE 2.A.
**2)** A T-Cell line CCRF-CEM cell culture containing 20x10⁶ is treated as described in section D.1.A.2. The cell pellet is resuspended in 200µl double distilled water, is homogenized with ultrasound and centrifuged as described in section D.1.B. The supernatant is collected, concentrated using centrifugation under vacuum up to 25µl volume and is analyzed using MALDI-TOF mass spectrometry as described in Section D.1.C. The corresponding spectrum is presented in FIGURE 2.B.
**3)** A T-Cell line CCRF-CEM cell culture containing 20x10⁶ is treated as describe in Section D.1.A.2. The cell pellet is resuspended in 200µl double distilled water, is homogenized with ultrasound and centrifuged as described in section D.1.B. The supernatant is collected (200µl) and diluted 3 times (600µl) with 100% acetonitrile, 0.1 % TFA and is recentrifuged at 15000g for 30 minutes at 4°C. The supernatant is concentrated using centrifugation under vacuum up to 25µl volume and is analyzed using MALDI-TOF mass spectrometry as described in Section D.1.C. The corresponding spectrum is presented in FIGURE 2.C.

From comparison of the mass spectrums presented in FIGURE 2, it is concluded that the use of 50% acetonitrile, 0.1% TFA aqueous solution made the selective NOP/SP isolation and further analysis with MALDI-TOF mass spectrometry possible.

### D.3. Conclusion

In the current invention an efficient method for the selective analysis of NOP/SP in cells and solid biological material was developed. The application of this method facilitated the release of the small protein molecules from carrier proteins (FIGURE 1) and their selective isolation from the proteins with molecular weight greater than 10kDa making their detection and identification with mass spectrometry possible.

### E. Advantages of the current invention

### E.1. NOP/SP isolation

As evident from FIGURES 1 and 2, the current method application seems to result in the NOP/SP separation from the rest of the solid biological material proteins with molecular weight greater than 10kDa unlike the rest of the methods which analyze NOP/SP as part of the low molecular weight proteome. The separation of the NOP/SPs from the rest of the solid biological material proteins constitutes the greatest advantage of the current method because after the separation, the molecules can be analyzed by all the available mass spectrometry technologies (MALDI-TOF, ESI-TOF etc.). A further advantage of the current method is that it does not include an enzymatic digest as a prerequisite for the NOP/SP identification. Because of that NOP/SP can be analyzed as naturally occurring molecules present in the solid biological materials and not as part larger protein molecules. The method application presented in the current invention can provide valuable information for the NOP/SP functionality in the biological fluids and contribute towards the investigation of low molecular weight biological markers related to various pathological conditions.

### E.2. MALDI-TOF analysis potential

In contradiction to the rest of NOP/SP analysis and identification methods, the method presented in the current invention provides the important potential for the analysis of these molecules with MALDI-TOF mass spectrometry. As mentioned above the particular technology is high throughput and combines high resolution with high intensity levels. The MALDI-TOF technology provides the potential for analysis of multiple samples since the analysis time per sample is less than a minute, the only disadvantage being that it is sensitive to contamination from organic and inorganic ions. This contamination affects the signal significance since a lot of background contaminant peaks are introduced in the spectrum. For the accurate evaluation of a signal the proteins being crystallized on the steel MALDI target must firstly be as contaminant-free as possible and secondly the NOP/SP must be present in such a quantity so as to provide a signal with at least double the noise intensity (signal/noise=2).

From the results acquired from the application of the specific method in a wide range of solid biological materials, it is evident that the quality and purity of NOP/SPs present in the sample that is spotted on the MALDI steel target is adequate to provide NOP/SP detection evaluation and identification. From the comparison of the current method with the rest of the methods (Chapter E.2.), it is conclude that the NOP/SP analysis method described in the current invention is the only one suitable for the analysis of these molecules with MALDI-TOF mass spectrometry.

### E.4. Rapid

The total time required for a full analysis of a sample with the method described in the current invention is approximately 1 hour in the case of which NOP/SP are analyzed with MALDI-TOF mass spectrometry. The 1 hour time is primarily required for sample preparation (centrifugation, concentration, desalting) and is substantially less than the time required by the methods currently used which include centrifugal ultrafiltration and enzymatic digest of the protein molecules prior to analysis. Furthermore because of the use of MALDI-TOF for NOP/SP analysis, the current method is substantially faster than the methods currently used since there is no sample chromatographic separation prior to mass spectrometry, which is a particularly time consuming step.

### E.5. High throughput

Because of the reliable NOP/SP analysis that can be performed with MALDI-TOF mass spectrometry, the invented method can be characterized as high throughput. The currently used methods for the NOP/SP analysis have the limitation of analyzing one sample at a time using liquid chromatography prior to mass spectrometry. The method described in the current invention, because of the MALDI-TOF analysis, provides the potential of analysis 384 sample in less than 2 hours.

### E.6. Subject to automation

A great advantage of the MALDI-TOF technology as compared to other mass spectrometry technologies is the potential for automation. This potential is provided by the use of specialized robotic systems for sample treatment and spotting on MALDI targets. By the use of these systems it is possible for 8 up to 384 samples to be treated and simultaneously spotted on a MALDI steel target. Because of the use of MALDI-TOF technology for the NOP/SP analysis, the method described in the current invention is subject to automation. In particular the tips used for the concentrated sample desalting and clean up can be placed on robotic systems in order for the sample desalting, spotting on the steel target and mixing with the matrix solution to be carried out automatically. Such robotic systems are already commercially available providing the capability of simultaneous handling of 8 to 384 samples and have been already widely utilized in parallel with the MALDI-TOF technology. The sample handling concerning its spotting on the steel target has to be particularly accurate since the volume being handled is very small and the sample positioning on the target is very tightly defined. The use of robotic systems would therefore facilitate the analysis reducing the preparation time and eliminating errors for a human operator.

### E.7. Large quantity of initial biological material and multiple simultaneous analysis potential.

Relevant studies have demonstrated that peptides and low molecular weight proteins originating in tissues cells or other solid state biological material can be analyzed utilizing mass spectrometry with minor or even no treatment by simply spotting the sample on the MALDI target and mixing it with matrix solution, or in the case of ESI-TOF by performing a simple buffer exchange in the infusion buffer (60% acetonitrile, 5% formic acid). [Baggerman G, Verleyen P, Clynen E, Huybrechts J, De Loof A, Schoofs L., Peptidomics J. Chromatography B 2004, 803:3-16]

Given the solid biological material complexity which might contain several hundreds or even thousands of different low molecular weight protein species, the analysis of the low molecular weight proteome by mass spectrometry adequate sample fractionation is required prior to mass spectrometric analysis. Furthermore, some of the protein species contained in the solid state biological material such as peptidic hormones or microbial toxins are of extremely low abundance, sample concentration is therefore required for their detection. Finally naturally occurring peptides and small proteins present in the solid state biological material are very often attached to high molecular weight, high abundance carrier proteins such as albumin. Therefore the application of methods which aim to remove the highly abundant proteins of the solid state biological material most probably result in the removal of the attached low molecular weight molecules which constitute potential biological markers.

The effectiveness of a method for the analysis of the solid biological material low molecular weight low abundance proteome must fulfill the following conditions: a) Sufficient separation of the small protein molecules from the carrier proteins, b) Sufficient separation of the NOP/SPs from the rest of the proteins present in the solid state biological material, c) Capability to analyze protein molecules present at low concentration.

The method presented in the current invention has the advantage of fulfilling all the above conditions. As evident from the results already presented (Chapter D.4.) NOP/SPs separated from the carrier proteins and from the rest of the proteins present in the biological material. Furthermore, the use of the MALDI-TOF technique which is characterized by high resolving power and high sensitivity, makes the highly accurate detection of NOP/SPs present in low concentrations possible.
**a)** Large initial solid state biological material volume treatment
   In the methods for the analysis of the solid biological material low molecular weight proteome currently in use, the initial solid biological material quantity used for the analysis is determined and restrained by the loading capacity of the ultracentrifugal filters and of the chromatography columns. The analysis of large quantities of solid biological material (eg. whole tissue) with these methods is either impossible or possible through sample aliquoting, simultaneous aliquot treatment and pooling back together prior to mass spectrometric analysis. The current method being independent from ultracentrifugal filtration and chromatography can be applied to any initial volume of biological materials which is treated and concentrated uniformly. This fact provides the potential for large volume treatment in order of the extremely low abundant NOP/SP to be detected and accurately identified.
**b)** Multiple simultaneous NOP/SP concentrated solution analysis
   From the current method application, the resulting NOP/SP concentrated solution can be further treated with different methods. Because of the high sensitivity of the mass spectrometry methods, the concentrated NOP/SP solution can be aliquoted and one aliquot to be analyzed with MALDI-TOF-MS, another with ESI-TOF-MS, another with µLC-MS, another one to be furtherly fractionated with liquid chromatography, another fraction to be analyzed by western blotting etc. The simultaneous concentrated NOP/SP solution analysis potential by different methods provides to the current method the advantage of thorough analysis of the biological fluid under study and the detection and identification of even the lowest abundance NOP/SPs.

### E.8. Quantification Potential

The method described in the current invention, opposing to the methods currently in use, possesses the advantage of NOP/SP quantification in the initial biological fluid, given the fact the concentrated NOP/SP solution can be analyzed with various methods. The quantitation can be achieved either by the use of sophisticated software performing signal analysis and protein profile comparison on the acquired spectra, or by the use of special modification reactions (isotopic labeling, adduct introduction reactions etc.), or by the use of internal standards. Thus, a defined quantity of one or more peptides of known molecular weight (Bradykinin and ACTH fragments) can be added to the concentrated or unconcentrated NOP/SP solution obtained during the stage described in section D.1.B. and after the procedure described in section D.1.C the peptides of the known molecular weight to be determined within the final NOP/SP mass spectrum. The NOP/SP quantitative determination in the initial biological material can be achieved through one of the quantitative analysis methods for an example through a standard curve of the internal standards correlating concentration to peak intensity and then exalting the NOP/SP concentrations based on than curve.

Internal standards can be added to other parts of the procedure apart from the concentrated or unconcentrated NOP/SP solution during the step described in Section D.1.B. in order for NOP/SPs to be quantitated such as to the matrix solution. The NOP/SP quantitation in the biological material can be achieved through one of the quantitative analysis methods (eg. standard curve, peak intensity comparison) by exaltation.

### E.9. Advantages summary

As opposed to the currently used methods for the solid biological material low molecular weight proteome analysis, the originality of the current invention lies to the fact that NOP/SPs are separated from the rest of the protein molecules with molecular weight greater than 10kDa and can be selectively analyzed using mass spectrometry as naturally occurring molecules.

The specific method does not include an enzymatic digest, nor any complicated chromatographic separations, is rapid, reproducible and subject to robotic automation. All these facts constitute the method described in the current invention as high throughput. The protein molecules separated with the use of this procedure can be detected, analyzed and identified with any of the available mass spectrometry methods including MALDI-TOF MS/MS without any limitations. Furthermore because of the low number of processing steps, the losses of the low abundant protein molecules present in the solid biological material is minimized.

### F. Figure Description

- **FIGURE 1.**: **A)** Representative image of an acrylamide gel containing the proteins of CCRF-CEM cells prepared according to the classic proteomic methodology as described in Section C.1.2.
**B)** Representative image of an acrylamide gel containing the proteins contained in the pellet of CCRF-CEM obtained after centrifugation of the cell homogenate in the acetonitrile/TFA solution, as described in Section D.2.A. **C)** Representative image of an acrylamide gel containing the proteins contained in the concentrated supernantant of CCRF-CEM obtained after centrifugation of the cell homogenate in the acetonitrile/TFA solution, as described in Section D.2.A. From the gel comparison it is concluded that by application of the method described in the current invention, the supernatant obtained after the centrifugation mostly contains naturally occurring peptides and small molecular weight proteins which have been selectively separated by proteins with molecular weight >10kDa. These proteins were collected in the pellet formed by the centrifugation of the cell homogenate.
- **FIGURE 2.**: **A)** T-cell line CCRF-CEM cell culture is treated as described in Sections D.1.A.2 and D.1.B, and NOP/SP are analyzed with MALDI-TOF mass spectrometry as described in section D.1.C. **B)** T-cell line CCRF-CEM cell culture is treated as described in Sections D.1.A.2. The cell pellet is resuspended in double distilled water, it is homogenized using ultrasound, is centrifuged as described in section D.1.B., the supernatant is collected, then concentrated under vacuum. NOP/SPs are analyzed using MALDI-TOF mass spectrometry as described in section D.1.C. **C)** T-cell line CCRF-CEM cell culture is treated as described in Sections D.1.A.2. The cell pellet is resuspended in double distilled water, it is homogenized using ultrasound, is centrifuged as described in section D.1.B. The supernantant is collected, diluted using 100% acetonitrile, 0.1%TFA and recentrifuged. The new supernatant is collected and concentrated using centrifugation under vacuum. It is then analyzed with MALDI-TOF mass spectrometry as described in section D.1.C.
- **FIGURE 3.**: Representative CCRF-CEM cells NOP/SP MALDI-TOF spectrum after treatment as described in section G.1 **A)** NOP/SP MALDI-TOF spectrum with molecular weight range 1-5kDa, **B)** NOP/SP MALDI-TOF spectrum with molecular weight range 1,6-4,9kDa. The arrows indicate the identified NOP/SPs which are presented in Table 1.
- **FIGURE 4.**: Representative NOP/SP MALDI-TOF spectrum from brain tissue after treatment as described in G.2. The arrows indicate the identified NOP/SPs which are presented in Table 1.
- **FIGURE 5.**: Representative NOP/SP MALDI-TOF spectrum from liver tissue after treatment as described in G.3. The arrows indicate the identified NOP/SPs which are presented in Table 1.

### G. IN DETAIL PRESENTATION OF A WAY TO APPLY THIS INVENTION

### G.1. T-cell line (CCRF-CEM) NOP/SP analysis using MALDI-TOF mass spectrometry.

For NOP/SP analysis using MALDI-TOF mass spectrometry, a human immature T-cell line (CCRF-CEM) was used. CCRF-CEM cell culture containing 20x10⁶ cells is centrifuged at 1200rpm for 10 minutes. The supernatant is disposed and the cell pellet is resuspended in 1ml ice cold normal saline, is transferred in a fresh eppendorf tube and is recentrifuged at 2200rpm for 10 minutes. The supernatant is disposed and the cell pellet is resuspended in 200µl 50% acetonitrile, 0.1%TFA aqueous solution and is vortexed for 10 minutes. The resuspended cells are homogenized by the use of ultrasound (ten 5sec cycles). The homogenate is centrifuged at 13000rpm (15000g) for 30 minutes at 4°C. After the end of the centrifugation the supernatant is collected and concentrated under vacuum up to a final volume of 20µl.

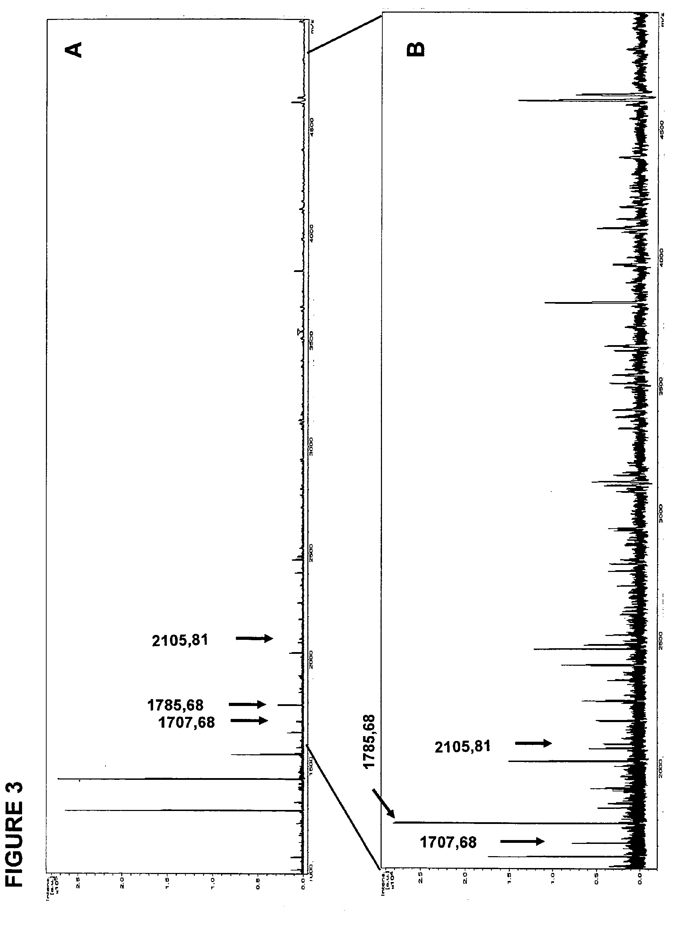

Prior to mass spectrometry analysis the sample is desalted using a pipette tip packed with C18 material. Prior to sample desalting the C18 material is washed 3 times by pipetting up and down 10µl of 100% acetonitrile in water and 3 times by pipetting up and down 10µl of 50% acetonitrile in water. Following that the material is equilibrating by 5 passes of 3% acetonitrile, 0.1%TFA solution (volume 10µl). The concentrated sample is pipetted up and down 30 times in order for the NOP/SP to be bound to the material. The bound NOP/SPs are then washed by 15 passes of 3% acetonitrile, 0.1%TFA solution (volume 10µl) and then eluted directly on the MALDI target using 3µl of 60% acetonitrile, 0.3%TFA in water. The eluted NOP/SPs are immediately mixed with 1µl of matrix solution [0.3%(w/v) a-Cyano-4-hydroxycinamic acid, 50%(v/v) acetonitrile, 0.1%(v/v)TFA] on target and are allowed to dry. After the mixture has dried, 1µl of matrix solution is added on top of the mixture and is again allowed to dry. After this procedure, the sample is placed in the mass spectrometer (MALDI ULTRAFLEX, Bruker Daltonics) for the analysis. The analysis is carried out for a molecular weight range of 1000-5200Da having the mass spectrometer set in reflector mode, the laser power at 80% and the detector gain set at 4. The final spectrum is the sum of 5 spectra each comprising 200 laser shots (total of 1000 laser shots). The obtained spectrum is presented in FIGURE 3. The spectrum analysis indicated that the peaks detected correspond to the analyzed human plasma NOP/SPs. The peptides with molecular weight 1707.68, 1785.68 and 2105.81 were identified by MS/MS sequence analysis. The results are presented on Table 1.

### G.2. Mouse brain NOP/SP analysis using MALDI-TOF mass spectrometry.

Mouse brain NOP/SPs were analyzed by MALDI-TOF mass spectrometry. In 100mg of mouse brain, 300µl 50% acetonitrile, 0.1%TFA aqueous solution is added and the mixture is vortexed for 10 minutes. Following that, the mixture is placed on ice and is homogenized initially by the use of a potter homogenator and finally by the use of ultrasound (ten 5sec cycles). The homogenate is centrifuged at 13000rpm (15000g) for 30 minutes at 4°C. After the end of the centrifugation the supernatant is collected and concentrated under vacuum up to a final volume of 30µl.

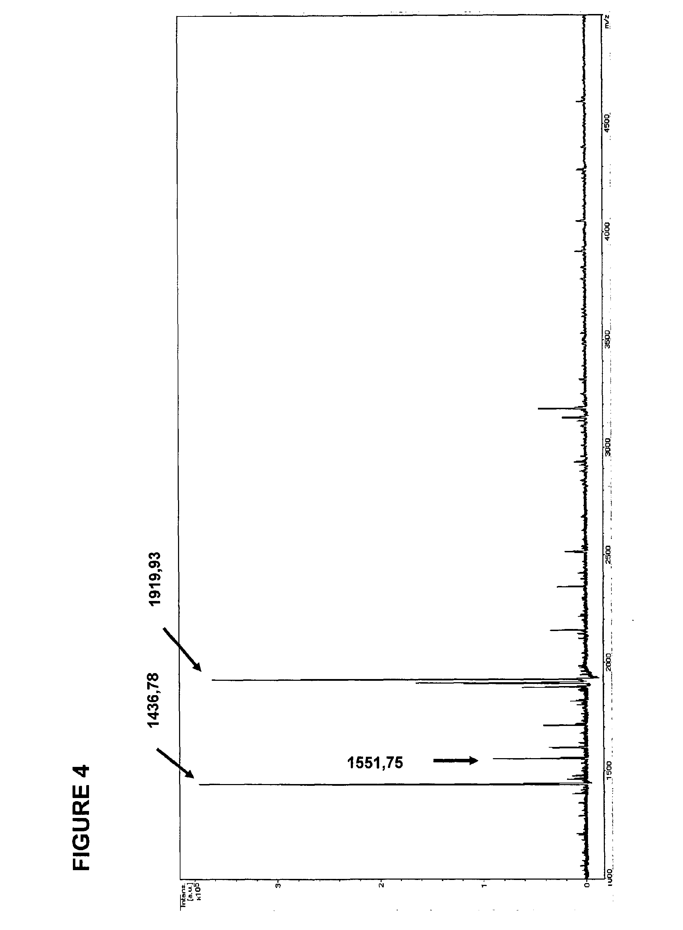

Prior to mass spectrometry analysis the sample is desalted using a pipette tip packed with C18 material. Prior to sample desalting the C18 material is washed 3 times by pipetting up and down 10µl of 100% acetonitrile in water and 3 times by pipetting up and down 10µl of 50% acetonitrile in water. Following that the material is equilibrating by 5 passes of 3% acetonitrile, 0.1%TFA solution (volume 10µl). The concentrated sample is pipetted up and down 30 times in order for the NOP/SP to be bound to the material. The bound NOP/SPs are then washed by 15 passes of 3%acetonitrile, 0.1%TFA solution (volume 10µl) and then eluted directly on the MALDI target using 3µl of 60%acetonitrile, 0.3%TFA in water. The eluted NOP/SPs are immediately mixed with 1µl of matrix solution [0.3%(w/v) a-Cyano-4-hydroxycinamic acid, 50%(v/v) acetonitrile, 0.1%(v/v)TFA] on target and are allowed to dry. After the mixture has dried 1µl of matrix solution is added on top of the mixture and is again allowed to dry. After this procedure the sample is placed in the mass spectrometer (MALDI ULTRAFLEX, Bruker Daltonics) for the analysis. The analysis is carried out for a molecular weight range of 1000-5200Da having the mass spectrometer set in reflector mode, the laser power at 80% and the detector gain set at 4. The final spectrum is the sum of 5 spectra each comprising 200 laser shots (total of 1000 laser shots). The obtained spectrum is presented in Figure 4. The spectrum analysis indicated that the peaks detected correspond to the analyzed human plasma NOP/SPs. The peptides with molecular weight 1436.78, 1551.75 and 1919.93 were identified by MS/MS sequence analysis. The results are presented on Table 1.

### G.3. Mouse liver NOP-SP analysis using MALDI-TOF mass spectrometry.

Mouse liver NOP/SPs were analysed by MALDI-TOF mass spectrometry. In 100mg of mouse liver, 300µl 50% acetonitrile, 0.1%TFA aqueous solution is added and the mixture is vortexed for 10 minutes. Following that, the mixture is placed on ice and is homogenized initially by the use of a potter homogenator and finally by the use of ultrasound (ten 5sec cycles). The homogenate is centrifuged at 13000rpm (15000g) for 30 minutes at 4°C. After the end of the centrifugation the supernatant is collected and concentrated under vacuum up to a final volume of 30µl.

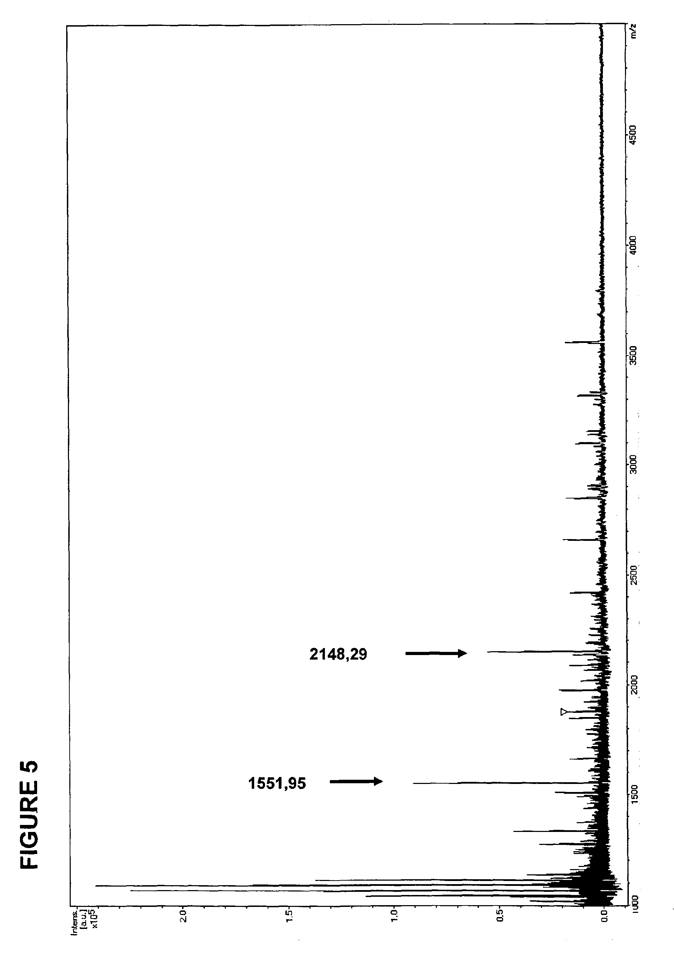

Prior to mass spectrometry analysis the sample is desalted using a pipette tip packed with C18 material. Prior to sample desalting the C18 material is washed 3 times by pipetting up and down 10µl of 100%acetonitrile in water and 3 times by pipetting up and down 10µl of 50%acetonitrile in water. Following that the material is equilibrating by 5 passes of 3%acetonitrile, 0.1%TFA solution (volume 10µl). The concentrated sample is pipetted up and down 30 times in order for the NOP/SP to be bound to the material. The bound NOP/SPs are then washed by 15 passes of 3%acetonitrile, 0.1%TFA solution (volume 10µl) and then eluted directly on the MALDI target using 3µl of 60%acetonitrile, 0.3%TFA in water. The eluted NOP/SPs are immediately mixed with 1µl of matrix solution [0.3%(w/v) a-Cyano-4-hydroxycinamic acid, 50%(v/v) acetonitrile, 0.1 %(v/v) TFA] on target and are allowed to dry. After the mixture has dried 1µl of matrix solution is added on top of the mixture and is again allowed to dry. After this procedure the sample is placed in the mass spectrometer (MALDI ULTRAFLEX, Bruker Daltonics) for the analysis. The analysis is carried out for a molecular weight range of 1000-5200Da having the mass spectrometer set in reflector mode, the laser power at 80% and the detector gain set at 4. The final spectrum is the sum of 5 spectra each comprising 200 laser shots (total of 1000 laser shots). The obtained spectrum is presented in Figure 5. The spectrum analysis indicated that the peaks detected correspond to the analyzed human plasma NOP/SPs. The peptides with molecular weight 1551.95 and 2148.29 were identified by MS/MS sequence analysis. The results are presented on Table 1.

**TABLE 1**

| | **MOLECULAR WEIGHT OF THE ANALYZED NOP/SP** | | | **SEQUENCE OF THE IDENTIFIED PEPTIDE** | **PROTEIN** | **PROTEIN SEQUENCE** |
|---|---|---|---|---|---|---|
| | **CCRF-CEM CELLS** | **MOUSE LIVER** | **MOUSE BRAIN** | | | |
| 1 | 1784.68 | | | IVNTNVPRASVPDGFLS | MIF_HUMAN (P14174) Macrophage migration inhibitory factor (MIF) | 1 PMFIVNTNVP RASVPDGFLS ELTQQLAQAT GKPPQYIAVH VVPDQLMAFG 51 GSSEPCALCS LHSIGKIGGA QNRSYSKLLC GLLAERLRIS PDRVYINYYD 101 MNAANVGWNN STFA |
| 2 | 2104.81 | | | ASSDIQVKELEKRASGQAF | STMN1_HUMAN, (P16949) Stathmin (Phosphoprotein p19) (pp19) (Oncoprotein 18) (Op18) (Leukemia-associated phosphoprotein) | 1 ASSDIQVKEL EKRASGQAFE LILSPRSKES VPEFPLSPPK KKDLSLEEIQ 51 KKLEAAEERR KSHEAEVLKQ LAEKREHEKE VLQKAIEENN NFSKMAEEKL 101 THKMEANKEN REAQMAAKLE RLREKDKHIE EVRKNKESKD PADETEAD |
| 3 | 1706.68 | | | PDDDDSCSINSDPEAK | Q51OG1_HUMAN, (Q5I0G1) APBB2 protein | 1 LKLRNAPHPD DDDSCSINSD PEAKCFAVRS LGWVEMAEED LAPGKSSVAV 51 NNCIRQLSYC KNDIRDTVGI WGEGKDMYLI LENDMLSLVD PMDRSVLHSQ 101 PIVSIRVWGV GRDNGRDFAY VARDKDTRIL KCHVFRCDTP AKAIATSLHE 151 ICSKIMAERK NAKALACSSL QERANVNLDV PLQVDFPTPK TELVQKFHVQ 201 YLGMLPVDKP VGMDILNSAI ENLMTSSNKE DWLSVNMNVA DATVTVISEK 251 NEEEVLVECR VRFLSFMGVG KDVHTFAFIM DTGNQRFECH VFWCEPNAGN 301 VSEAVQAACM LRYQKCLVAR PPSQKVRPPP PPADSVTRRV TTNVKRGVLS 351 LIDTLKQKRP VTEMP |
| 4 | | | 435.76 | NNGKAEFWLDLQ | Q6ZMP3_HUMAN (Q6ZMP3) Hypothetical protein FLJ16784 | 1 MKEALSTERG KTLVQKKPTM YPEWKSTFDA HIYEGRVIQI VLMRAAEEPV 51 SEVTVGVSVL AERCKKNNGK AEFWLDLQPQ AKVLMSVQYF LEDVDCKQSM 101 RSEDEAKFPT MNRRGAIKQA KIHYIKNHEF IATFFGQPTF CSVCKDFVWG 151 LNKQGYKCRR KCLHRPVCTY VPMCAHVCQC LCVCQCLCAL RECMHVSFPS 201 VATAAEFSEC WGWLAAALEN QFCASLPSSA FSDGELVDGN WAQ |
| 5 | | | 550.75 | QLGARACATGSDLTST | Q9BFV4_MYRTR. (Q9BFV4) Amyloid beta protein | 1  |
| 6 | | | 918.93 | LKKCGDLECETLISRVL | i MIA2_MOUSE, (Q91ZVO) Melanoma inhibitory activity protein 2 | 1 MAEVSVQRIL LLVVSLAKCL EGTKLLAHLK KCGDLECETL ISRVLALRDY 51 TGPDCRYLNF TTGEEISVYV KLGGDREDLW AGSKGKDFGF FPRDAVEIEE 101 VFISEEVEMP TKSDFLCLLG EGYIFGSEQS ELNSEDDEEH MYPYEKDEDQ 151 NYNIYEGDFQ PEPDLYAAAE GTLLEDQIPA SEAPDDFRFS SEWKAWEGAG 201 SQGGGEQDYT ADSDQDLPSL SKPERQGWFG LGTEEAEEKV FESDTEPTQE 251 LALEEESDLE KLHSGEPQVE LEQEPKSETL EFSSVPDEEY ELESETESIL 301 KPQASGWFGE GLTSYLGFGN EEAGLELLSK ESNPPLQDIP SSVPPDEEVP 351 APCREISTDK EDAVINDSSV LSPSWFYYGF GMLGFTNADE DNIVSDKGEN 401 EDGEVDNLKH PIGSDFDPEK EQERKIVTVE TEDQAGTESV LEKTDESGSM 451 QYLKKFFDNP WGFQSLPEDT ELPFSKKMLD QDDIVENDKI EELSTENSPT 501 GSMKDPVMLA SRYVLSG |
| 7 | | 1550.95 | | MASSTPSPATSSNAGAD | Q76N58_MOUSE (Q76N58) Glutamic acid decarboxylase 67 | **1 MASSTPSPAT SSNAGAD** |
| 8 | | 2147.29 | | MAALDSKASGKMGMRAVV | Q8C7W8_MOUSE (Q8C7W8) | 1 MTKSNGEEPR MGGRMERLQQ GVRKRTLLAK KKVQSLTKED VKSYLFRNAF 51 VLLTVTAVIV GTILGFALRP YKMSYREVKY FSFPGELLMR MLQMLVLPLI 101 ISSLVTGMAA LDSKASGKMG MRAVVYYMTT TIIAVVIGII IVIIIHPGKG 151 TKENMYREGK IVQVTAADAF LDLIRNMFPP NLVEACFKQF KTSYEKRSFK 201 VPIQSNETLL GAVINNVSEA METLTRIREE MVPVPGSVNG VNALGLVVFS 251 MCFGFVIGNM KEQGQALREF FDSLNEAIMR LVAVIMWYAP LGILFLIAGK 301 IVEMEDMGVI GGQLAMYTVT VIVGLLIHAV IVLPLLYFLV TRKNPWVFIG 351 GLLQALITAL GTSSSSATLP ITFKCLEENN GVDKRITRFV LPVGATINMD 401 GTALYEALAA IFIAQVNNFD LNFGQIITIS ITATAASIGA AGIPQAGLVT 451 MVIVLTSVGL PTDDIALIIA VDWFLDRLRT TTNVLGDSLG AGIVEHLSRH 501 ELKNRDVEMG NSVIEENEMK KPYQLIAQDN EPEKPVADSE TKM |

## Claims

1. One method for the analysis of one or more or all of the protein molecules of a solid biological material comprising of: **a)** The mixing or the addition of the solid biological material with aqueous solution of 50% acetonitrile containing 0.1%TFA, **b)** the homogenization of the mixture, **c)** its centrifugation, **d)** the separation of the supernatant and its concentration, **e)** its desalting and cleaning, **f)** its analysis with mass spectrometry.

2. One method where according to Claim 1, the solid biological material is a tissue or a tissue section or biopsy sample or sample that is obtained by invasive or non-invasive methods (surgery, biopsy, amniocentesis etc).

3. One method where according to Claim 1, the solid biological material is cells surviving in cultures or obtained by cell culture handling and/or cell parts, subcellular fractions, cellular organelles etc.

4. One method where according to Claim 1, the solid biological material is cell or cell species or cell sections originating from tissues or biological fluids (peripheral blood, amino fluid, urine, etc).

5. One method according to Claims 1-4, where the protein molecules have a molecular weight less than 10kDa.

6. One method where, according to Claims 1-5, the protein molecules belong to the group of the Naturally Occurring Peptides and Small Proteins.

7. One method where, according to claim 1, the homogenization is carried out mechanically or by the use of ultrasound, or liquid nitrogen etc. or by a combination of these methods.

8. One method where according to Claim 1, the solutions (supernatant or concentrated solution) of stage (d) of the method, are furtherly processed.

9. One method, where according to Claim 1, the solution obtained after the end of stage (d) of the method, is desalted and cleaned with the use of an appropriate retention material.

10. One method where, according to Claim 9, the tip retention material is type C18.

11. One method where according to Claim 8, the solutions of the stage (d) of the method (supernatant or concentrated solution) are furtherly separated in fractions.

12. One method where according to Claim 11, the fractionation is carried out using chromatography.

13. One method where according to Claims 1 - 6, the analysis includes the separation and/or the identification and/or the qualitative specification and/or the quantitative specification of one or more or all of the protein molecules.

14. One method where according to Claim 13, the quantitative specification of one or more or all of the protein molecules is carried out using one or more internal controls.

15. One method where according to Claims 13 and 14, the quantitation is carried out using specialized algorithms.

16. One method where according to Claim 1, the mass spectrometry technology is MALDI-TOF, SEDLI-TOF, ESI-TOF, ESI-IT, ESI-FT, ESI-QqTOF, ESI-QqQ etc.

17. One method where according to Claim 1, the whole procedure or part of it, is carried out by robotic systems.

18. One method where according to Claims 1 to 8 and 13, one or more or all of the protein molecules in the solutions of the stage (d) of the method are analyzed with a method different from mass spectrometry.

19. One method, according to Claims 1 to 18, for the detection and analysis of biological markers indicative of pathological conditions.

20. One method, according to Claims 19 where the biological markers are one or more molecules from the group of naturally occurring peptides and/or small proteins.

21. One method, according to Claims 1 to 20, for the diagnosis of pathological conditions through comparison, evaluation and/or analysis of protein patterns and/or profiles.

22. The use of the method, according to claims 1 to 21, for the analysis of one or more protein molecules.

23. The use of the method, according to claims 1 to 22, for the analysis of one or more molecules from the group of naturally occurring peptides and/or small proteins.

24. The use of the method, according to Claims 1 to 23, for the detection and analysis of biological markers.

25. The use of the method, according to Claims 1 to 24, for the diagnosis of pathological conditions.

26. The use of the aqueous solution containing 50% acetonitrile and 0.1% TFA in the process of solid biological materials as homogenization solution.

27. The use of the acetonitrile-TFA solution, where according to Claim 26, aims at the analysis of one or more protein molecules.

28. The use of the acetonitrile-TFA solution, where according to Claim 27, the analysis includes the detection and/or the separation and/or the identification and/or the qualitative and/or the quantitative determination.

29. The use of the acetonitrile-TFA solution, according to Claims 27 and 28, to the analysis of one or more molecules from the group of the naturally occurring peptides and/or small proteins.

30. The use of the acetonitrile-TFA solution, according to Claims 26 to 29, where the solid state biological material is tissue or tissue section or biopsy sample or sample obtained by invasive or non-invasive methods (surgery, amniocentesis, etc.) or cells or cell fractions from cells surviving in cell culture or cells originating from cell culture handling, or cells, cell species or cell sections originating from tissues or biological fluids (peripheral blood, amniotic fluid, urine, etc.).

31. The use of the acetonitrile-TFA solution, according to Claims 26 to 30, where the analysis is carried out with mass spectrometry.

32. The use of the acetonitrile-TFA solution, according to Claims 26 to 31, to the analysis of biological markers.

33. The use of the acetonitrile-TFA solution, according to Claims 26 to 32, to the diagnosis of pathological states.

34. A product for the analysis of one or more or all of the protein molecules, which are contained in solid biological materials and is **characterized by** mixing aqueous solution containing 50% acetonitrile and 0.1% TFA with the solid biological material, the mixture's homogenization, its centrifugation, the supernatant separation, its concentration, desalting and cleaning and its analysis with mass spectrometry.

35. A product where according to Claim 34, the analysis includes the separation and/or the identification and/or the qualitative specification and or the quantitative specification one or more or all of the protein molecules.

36. A product where according to Claims 34 and 35, the mass spectrometry technology is MALDI-TOF, SEDLI-TOF, ESI-TOF, ESI-IT, ESI-FT, ESI-QqTOF, ESI-QqQ etc.

37. A product where according to Claims 34 to 36, the protein molecules have molecular weight less than 10kDa.

38. A product where according to Claim 37, the molecules belong to the group of naturally occurring peptides and/or small proteins.

39. A product, according to Claims 34 to 36, for the detection and analysis of biological markers.

40. A product, where according to Claim 39, the biological markers are one on more molecules belonging to the group of the naturally occurring peptides and/or small proteins.

41. A product, according to Claims 34 to 40, for the diagnosis of pathological states.

42. A product, according to Claims 34 to 41, for the diagnosis of pathological states through protein patterns and/or profiles.

43. A product for the treatment of solid biological material, which is **characterized by** the use of aqueous solution containing 50% acetonitrile and 0.1 % TFA.

44. A product where, according to Claim 43, the solid state biological material is tissue or tissue section or biopsy sample or sample obtained by invasive or non-invasive methods (surgery etc.) or cells or cell sections that survive in cell culture or cells obtained from culture handling or cells or cell sections obtained from tissues or biological fluids (peripheral blood, amniotic fluid, urine etc.)

45. A product which, according to Claims 43 and 44, aims at the analysis one or more or all protein molecules in a solid state biological material.

46. A product where according to Claim 45, the molecules belong to the group of naturally occurring peptides and/or small proteins.
